# FASCICULE DE BREVET EUROPEEN

(11) **EP 1 986 712 B1**
(45) Date de publication et mention de la délivrance du brevet: **23.12.2015**
(21) Numéro de dépôt: 07731017.5
(22) Date de dépôt: 21.02.2007
(51) Int. Cl.: A61L 27/34

(54) **UTILISATION DE FUCANES A DES FINS DE GREFFE, D'INGENIERIE ET DE REGENERATION OSSEUSES**
VERWENDUNG VON FUCANEN ZUM ZWECKE DER KNOCHENTRANSPLANTATION, -KONSTRUKTION UND -REGENERATION
USE OF FUCANS FOR THE PURPOSES OF BONE GRAFTING, ENGINEERING AND REGENERATION

(30) Priorité: 23.02.2006 FR 0601618
(43) Date de publication de la demande: 05.11.2008
(73) Titulaire: Université René Descartes Paris 5, 72270 Paris Cedex 06 (FR); Institut Francais de Recherche pour l'Exploitation de la Mer (IFREMER), 92130 Issy-les-Moulineaux Cedex (FR)
(72) Inventeur: IGONDJO-TCHEN CHANGOTADE, Sylvie, F-77500 Chelles (FR); SENNI, Karim, F-93600 Aulnay sous Bois (FR); FOUCAULT-BERTAUD, Alexandrine, F-13004 Marseille (FR); KORB, Grégory, F-94300 Vincennes (FR); BAROUKH, Maya, Brigitte, F-75014 Paris (FR); SAFFAR, Jean-Louis, F-92120 Montrouge (FR); GODEAU, Gaston-Jacques, F-92160 Antony (FR); SINQUIN, Corinne, F-44300 Nantes (FR); COLLIEC-JOUAULT, Sylvia, F-44100 Nantes (FR); DURAND, Patrick, F-44400 Reze (FR)
(74) Mandataire: Cabinet Plasseraud
(86) Numéro de dépôt international: PCT/FR2007/000310
(87) Numéro de publication internationale: WO 2007/096519

(56) Documents cités:
- WO-A2-02/02051
- WO-A2-99/32099
- KARIYA Y ET AL: "Isolation and partial characterization of fucan sulfates from the body wall of sea cucumber Stichopus japonicus and their ability to inhibit osteoclastogenesis" CARBOHYDRATE RESEARCH, ELSEVIER SCIENTIFIC PUBLISHING COMPANY. AMSTERDAM, NL, vol. 339, no. 7, 17 mai 2004 (2004-05-17), pages 1339-1346, XP004504518 ISSN: 0008-6215
- BERTEAU O ET AL: "Sulfated fucans, fresh perspectives: Structures, functions, and biological properties of sulfated fucans and an overview of enzymes active toward this class of polysaccharide" juin 2003 (2003-06), GLYCOBIOLOGY, IRL PRESS,, GB, PAGE(S) 29R-40R , XP002338357 ISSN: 0959-6658 cité dans la demande figure 1

## Description

La présente invention se rapporte à l'utilisation de fucanes de masse molaire moyenne en poids comprise entre 5000 et 100 000 g/mol à des fins de greffe, d'ingénierie et de régénération osseuses.

Les fucanes sont une famille de polysaccharides sulfatés (cf. Berteau et Mulloy, Glycobiology, vol 13, no 6, pp 29R-40R, 2003). Les fucanes sont des polysaccharides constitués majoritairement de chaînes d'unités de fucose et riches en sulfate (homofucane) et de chaînes moins sulfatées composées d'unités de fucose, galactose, xylose et d'acide glucuronique (xylofucoglucuronanes). Les fucanes peuvent être extraits à partir des algues, notamment à partir d'algues brunes (Phéophycée) ou à partir d'invertébrés marins. Les fucanes, une fois extraits, présentent généralement une masse molaire moyenne en poids supérieure à 500 000 g/mol. De nombreuses techniques existent pour dépolymériser les fucanes afin d'obtenir des fucanes présentant une masse molaire moyenne en poids comprise entre 5 000 et 100 000 g/mol (cf. EP0403377) De nombreuses applications thérapeutiques des fucanes ont été décrites : anticoagulant (cf. EP0403377), traitement des pathologies parodontales et des lésions dermiques (cf. WO9932099), antithrombotique (cf. WO011565), traitement de l'arthrite et de l'ostéoarthrite (cf. WO03018033).

Le comblement des pertes de substance osseuse est un problème fréquemment rencontré dans les pathologies et les traumatismes ostéoarticulaires (tumeurs osseuses, séquelles traumatiques, pathologies dégénératives). Le cahier des charges d'un matériau de remplacement osseux idéal inclut en particulier : 1) une bonne tolérance sans risques infectieux pour le receveur 2) une intégration aux structures osseuses *in situ* 3) une capacité à permettre une néoformation osseuse de bonne qualité dotée de propriétés mécaniques suffisantes pour supporter les contraintes au niveau du site d'implantation. Parmi ces matériaux des biomatériaux naturels ou de synthèse constituent l'alternative aux greffes osseuses. Les propriétés recherchées pour ces matériaux sont l'ostéoconduction c'est-à-dire la capacité à guider la reconstruction osseuse à leur contact et/ou l'ostéoinduction c'est à dire la capacité à stimuler la formation osseuse même en site extraosseux. Plusieurs voies de recherche (cf. Heymann et al. Revue de chirurgie orthopédique, 2001 ; 87, 8-17) tendent à améliorer les propriétés ostéogènes des biomatériaux. L'association aux biomatériaux de protéines de la matrice extracellulaire ou de facteurs de croissance à potentiel ostéogène est un des axes de recherche. Ces facteurs de croissance peuvent être incorporés aux biomatériaux et relargués *in vivo* potentialisant ainsi l'ostéogénèse au sein de ceux-ci. Les céramiques en phosphate de calcium (PCa) ont été également considérées comme des systèmes de délivrance de substances pharmacologiques (anti-cancéreux, antibiotiques...). L'association de cellules ostéocompétentes, c'est-à-dire des cellules capables de constituer du tissu osseux, aux substituts osseux est l'autre voie de recherche principale. Cette association « hybride » nécessite un substitut de comblement d'une part et une source autologue de cellules ostéocompétentes d'autre part. Ces cellules peuvent provenir d'explants osseux à partir desquels les cellules ostéoblastiques sont isolées. Cette méthode ne permet cependant que d'obtenir une faible quantité de cellules ostéocompétentes. Les cellules ostéocompétentes peuvent également provenir d'ostéoprogéniteurs médullaires capables de se différencier *in vitro* et *in vivo.* A ce titre l'ensemble des cellules de moelle ou seulement les cellules stromales peuvent être utilisés. Il est encore possible d'obtenir un clone ostéoblastique différencié à partir des cellules progénitrices de la moelle. Des cellules de moelle osseuse injectées en site osseux peuvent à elles seules combler un déficit osseux calibré chez le rat. L'association de cellules de moelle osseuse à une céramique en PCa permet à la céramique d'acquérir un caractère ostéoconducteur. En effet de nombreux travaux réalisés chez l'animal mettent en évidence une ossification au sein de céramiques incubées avec des cellules de moelle osseuse autologue puis implantées en site extra-osseux. De plus les cellules de moelle osseuse potentialisent l'intégration du biomatériau en site osseux. Elles favorisent la rapidité et la qualité de la repousse osseuse.

WO0202051 décrit l'utilisation d'un polysaccharide excrété par l'espèce *Vibrio Diabolicus* en cicatrisation osseuse. Le polysaccharide peut être utilisé pour la préparation d'un revêtement d'un implant endoosseux ou d'un matériau de comblement ostéoconducteur. Selon l'enseignement de WO0202051, les fucanes présentant un haut poids moléculaire (de l'ordre de 1 million de g/mol) n'ont aucun effet sur la cicatrisation osseuse.

Au vu des insuffisances et des inconvénients de l'état de la technique en matière de reconstruction osseuse, les Inventeurs se sont donnés pour but de pourvoir à un substitut osseux aux propriétés ostéoconductrices améliorées.

De façon surprenante, les Inventeurs ont découvert que ce but pouvait être atteint en utilisant une famille de polysaccharides particulière, à savoir les fucanes de masse molaire moyenne en poids comprise entre 5 000 et 100 000 g/mol. Ces fucanes sont capables de promouvoir et d'accélérer, la migration, la prolifération des cellules ostéoblastiques, ainsi que la synthèse d'une nouvelle matrice extracellulaire osseuse et sa minéralisation.

L'invention a pour objet un substitut osseux comprenant un biomatériau et des fucanes présentant une masse molaire moyenne en poids comprise entre 5 000 et 100 000 g/mol, en particulier comprise entre 10 000 et 40 000 g/mol.
Les fucanes utilisés dans la présente invention présentent une masse molaire moyenne en poids comprise entre M1 et M2, les masses molaires moyennes en poids M1 et M2 étant choisies indépendamment l'une de l'autre, M1 étant choisie parmi les valeurs 5 000, 10 000, 15 000, 20 000 et 25 000 g/mol et M2 étant choisie parmi les valeurs 40 000, 60 000, 80 000, 90 000 et 100 000 g/mol.
De manière préférentielle les fucanes sont obtenus à partir d'algue brune. L'homme du métier pourra utiliser les techniques usuelles pour obtenir les fucanes utilisés dans la présente invention. On peut citer à titre d'exemple de techniques utilisables : par la lyse ménagée des fucanes de haut poids moléculaire suivie d'une purification par chromatographie sur gel comme précédemment décrit (cf. EP0403377) ou par la dépolymérisation radicalaire des fucanes de haut poids moléculaire comme décrit précédemment (EP0846129 ou WO9708206), l'homme du métier adaptera facilement les conditions opératoires afin d'obtenir la masse molaire moyenne en poids souhaitée.

Par biomatériau, on entend tout matériau voué au remplacement d'une fonction ou d'un organe. Il s'agit de tout matériau non vivant utilisé dans un dispositif médical et visant à remplacer ou traiter un tissu, un organe ou une fonction.

Typiquement, pour préparer un substitut osseux selon l'invention, l'homme du métier pourra utiliser tout biomatériau couramment utilisé en ingénierie osseuse. A titre d'exemple, le biomatériau pourra comprendre un ou plusieurs matériaux choisis dans le groupe constitué par le titane, le collagène, l'os déprotéinisé et/ou déminéralisé, le corail, la céramique en phosphate de calcium, l'hydroxyapatite, le phosphate tricalcique beta et les verres bioactifs.

Avantageusement, un substitut osseux selon l'invention pourra comprendre en outre un ou plusieurs facteurs de croissance choisis dans les groupes comprenant les Fibroblast Growth Factors (FGFs), les Transforming Growth Factors (TGFs), les Insulin Growth Factors I (IGFs), les Platelet Derived Growth Factors (PDGFs) et les Bone Morphogenetic Proteins (BMPs), les Vascular Endothelial Growth Factors (VEGFs).
Préférentiellement, un substitut osseux selon l'invention pourra comprendre en outre un ou plusieurs facteurs de croissance choisi (s) dans le groupe constitué par BMPs, FGFs, TGF beta et VEGFs.
Avantageusement un substitut osseux selon l'invention pourra comprendre en outre une ou plusieurs cytokines choisie(s) dans le groupe constitué par l'interleukine 1, interleukine 6, l'interleukine 4, Tumor Necrosis Factor alpha, Granulocyte-Macrophage Colony-Stimulating Factor et Macrophage Colony-Stimulating Factor.

Typiquement, la surface du biomatériau d'un substitut osseux selon l'invention présente un revêtement comprenant les fucanes.
L'homme du métier pourra utiliser les techniques usuelles pour recouvrir la surface du biomatériau par un revêtement comprenant les fucanes. A titre d'exemple, l'homme du métier pourra greffer les fucanes sur la surface du biomatériau.

Le biomatériau d'un substitut osseux selon l'invention pourra également être imprégné avec les fucanes.

Avantageusement, un substitut osseux selon l'invention pourra comprendre en outre des cellules choisies dans le groupe constitué des cellules ostéocompétentes issues de la moelle osseuse, d'explants osseux ou périostés.
Pour préparer de tels substituts osseux, l'homme du métier pourra procéder à la colonisation d'un substitut osseux par les cellules souhaitées.

Selon un mode de réalisation, l'invention a trait à un milieu de culture pour un type de cellules choisi dans le groupe constitué des cellules ostéocompétentes issues de la moelle osseuse, de l'os ou du périoste, comprenant des fucanes présentant une masse molaire moyenne en poids comprise entre 5 000 et 100 000 g/mol, en particulier comprise entre 10 000 et 40 000 g/mol.

Typiquement un milieu de culture selon l'invention pourra comprendre en outre un ou plusieurs facteurs de croissance choisis dans le groupe constitué par les Fibroblast Growth Factors (FGFs), les Transforming Growth Factor (TGFs), les Insulin Growth Factors I (IGFs), les Platelet Derived Growth Factors (PDGFs) et les Bone Morphogenetic Proteins (BMPs) et les Vascular Endothelial Growth Factors (VEGFs). Selon un mode de réalisation, l'invention a trait à l'utilisation des lucanes présentant une masse molaire moyenne en poids comprise entre 5 000 et 100 000 g/mol, en particulier comprise entre 10 000 et 40 000 g/mol pour la culture de cellules ostéocompétentes issues de la moelle osseuse, de l'os ou du périoste.

Selon un mode de réalisation, l'invention a trait à un procédé de culture de cellules choisies dans le groupe des cellules ostéocompétentes issues de la moelle osseuse, de l'os ou du périoste caractérisé en ce que lesdites cellules sont cultivées dans un milieu de culture décrit précédemment.

Selon un mode de réalisation, l'invention a trait à un procédé de culture de cellules ostéocompétentes issues de la moelle osseuse, de l'os ou du périoste, caractérisé en ce que lesdites cellules sont cultivées sur un substitut osseux décrit précédemment.

Selon un mode de réalisation, l'invention a trait à l'utilisation des fucanes présentant une masse molaire moyenne en poids comprise entre 5 000 et 100 000 g/mol, en particulier comprise entre 10 000 et 40 000 g/mol pour la fabrication d'un dispositif médical présentant une activité sur la régénération osseuse.

Typiquement un tel dispositif médical pourra être utilisé en chirurgie orthopédique, parondontale et plastique afin de faciliter la régénération osseuse et de combler les défauts tissulaires survenant, par exemple, après une lésion due à une pathologie, à un accident ou à un acte chirurgical.

Un tel dispositif médical pourra, par exemple, être utilisé comme matériau de réparation ou de comblement osseux, comme greffon osseux, comme implant endo-osseux, comme implant dentaire ou ostéoarticulaire.
Un tel dispositif médical pourra servir au traitement de pathologies osseuses comme, par exemple, l'ostéoporose.
Un tel dispositif médical pourra, par exemple, comprendre les fucanes, préférentiellement sous une forme hydratée, par exemple sous la forme d'un hydrogel, seul ou en combinaison avec un ou plusieurs facteurs de croissance choisis dans le groupe constitué par les Fibroblast Growth Factors (FGFs), les Transforming Growth Factors (TGFs), les Insulin Growth Factors I (IGFs), les Platelet Derived Growth Factors (PDGFs) et les Bone Morphogenetic Proteins (BMPs), les Vascular Endothelial Growth Factors (VEGFs). L'association des fucanes à un facteur de croissance permet d'augmenter la vitesse de la régénération osseuse. Préférentiellement, un dispositif médical selon l'invention comprendra des fucanes sous une forme hydratée et un ou plusieurs facteurs de croissance choisi(s) dans le groupe des BMPs, FGFs, TGF beta et VEGFs.

Typiquement un dispositif médical selon l'invention pourra comprendre un substitut osseux selon l'invention.

Le contenu de tous les documents cités doit être considéré comme faisant partie de la présente description.

La présente invention sera mieux illustrée ci-après à l'aide des exemples qui suivent. Ces exemples sont donnés uniquement à titre d'illustration de l'objet de l'invention, dont ils ne constituent en aucune manière une limitation.

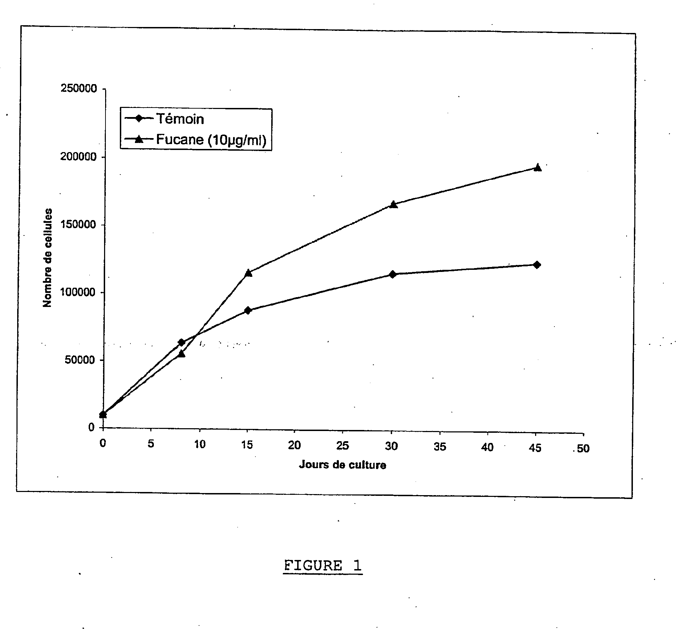

Là figure 1 présente l'évolution du nombre d'ostéoblastes en fonction du temps en présence et en absence des fucanes.

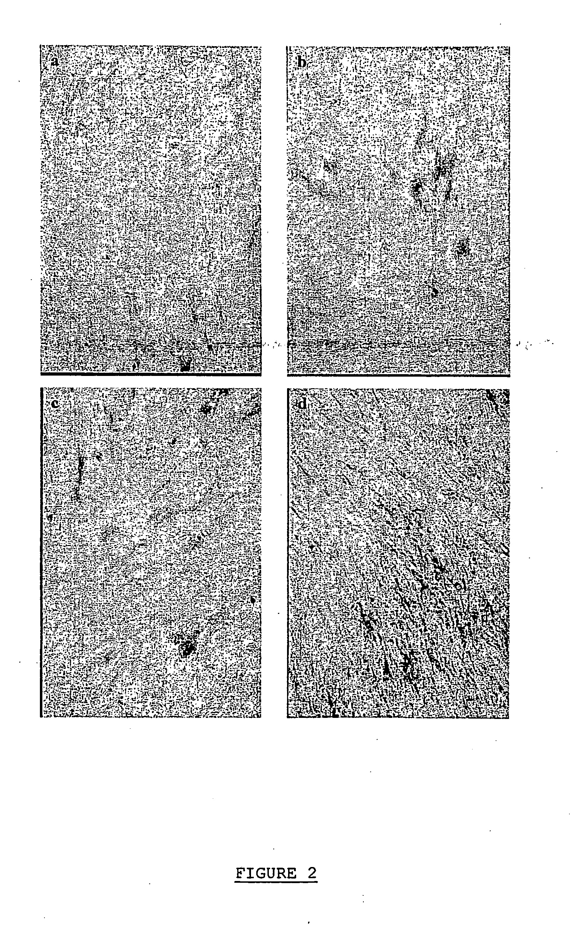

Les figures 2a, 2b, 2c, 2d représentent des photographies de culture de cellules ostéoblastiques témoin sur lesquelles l'activité phosphatase alcaline est détectée.
Les figures 2a, 2b, 2c et 2d représentent des photographies réalisées après 8, 15, 30 et 45 jours de culture respectivement.

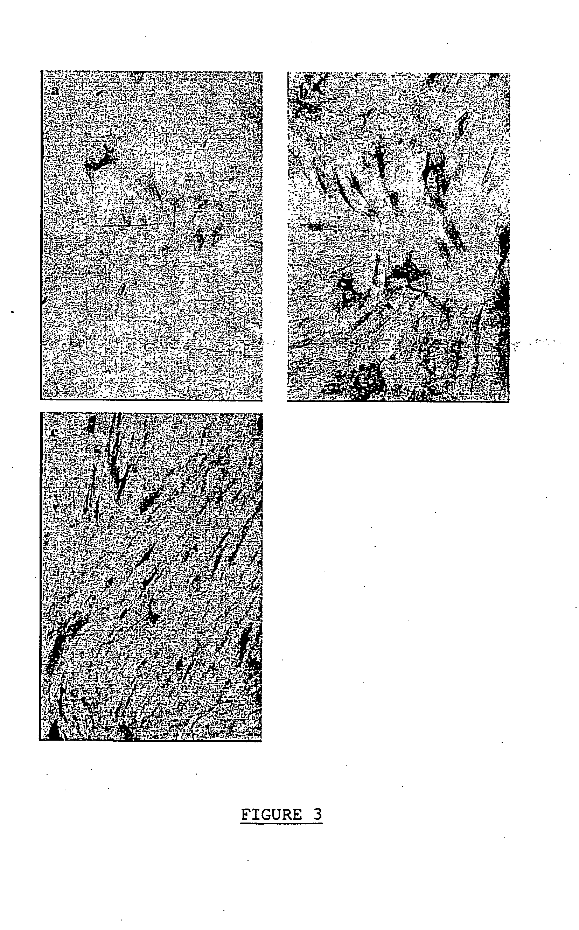

Les figures 3a, 3b, 3c représentent des photographies de culture de cellules ostéoblastiques faite en présence de 10 µg/ml de fucanes. L'activité phosphatase alcaline a été détectée en final sur les cellules en culture. Les figures 3a, 3b, et 3c représentent des photographies réalisées après 8, 15 et 30 jours de culture respectivement.

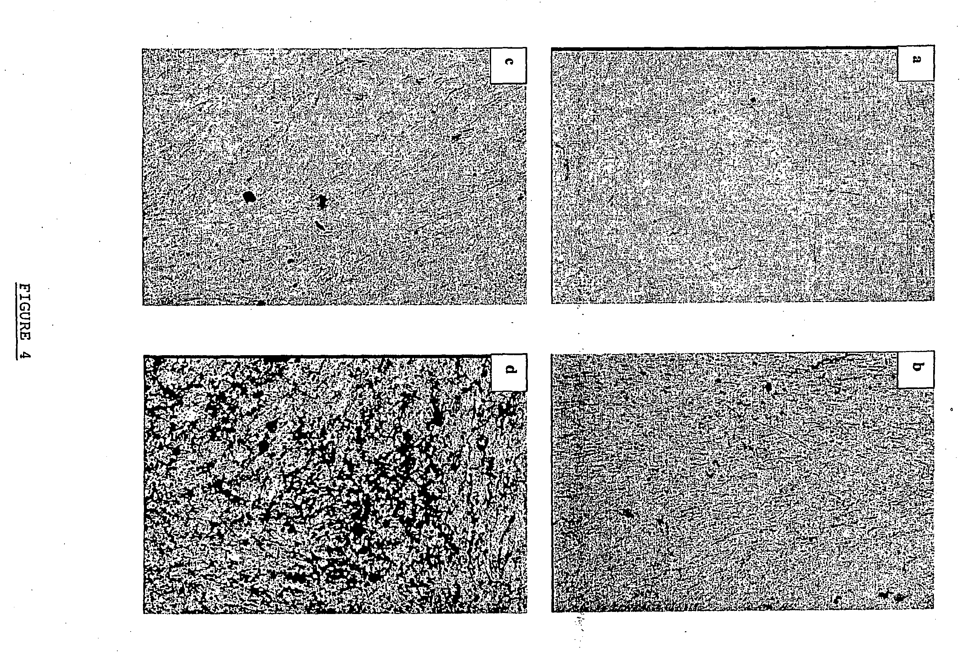

Les figures 4a, 4b, 4c, 4d représentent des photographies de culture de cellules ostéoblastiques témoin (figures 4a et 4c) et de culture de cellules ostéoblastiques faite en présence 10 µg/ml de fucanes (figures 4b et 4d). Les dépôts minéraux sont visualisés par la réaction de Von Kossa réalisée en final après fixation des cellules en culture.
Les figures 4a et 4b représentent des photographies réalisées après 30 jours de culture. Les figures 4c et 4d représentent des photographies réalisées après 45 jours de culture.
Les figures 5a, 5b représentent des photographies de culture de cellules ostéoblastiques témoin (figure 5a) et de cultures de cellules ostéoblastiques en présence de 10 µg/ml de fucanes (figure 5b). Après 45 jours de culture, l'immuno-détection du collagène de type I est réalisée sur les cellules en culture.
Les figures 6a et 6b représentent des images réalisées par microscopie électronique à balayage (MEB) de biomatériaux acellulaires témoins (figure 6a) ou imprégnés de fucanes (figure 6b).
Les figures 7a et 7b représentent des images réalisées par microscopie électronique à balayage (MEB) de biomatériaux témoins (figure 7a) ou imprégnés de fucanes (figure 7b) sur lesquels des ostéoblastes ont été cultivés pendant 10 jours.
Les figures 8a et 8b représentent des images réalisées par microscopie électronique à balayage (MEB) de biomatériaux témoins (figure 8a) ou imprégnés de fucanes (figure 8b) sur lesquels des ostéoblastes ont été cultivés pendant 30 jours
La figure 9 représente montre la profondeur des biomatériaux imprégnés par les fucanes sur lesquels des ostéoblastes ont été cultivés pendant 30 jours.
Les figures 10a et 10b représentent des images réalisées par microscopie électronique à balayage (MEB) de biomatériaux témoins (figure 10a) ou imprégnés de fucanes (figure 10b) sur lesquels des ostéoblastes ont été cultivés pendant 10 jours et montrant la matrice extracellulaire sécrétée par les cellules

### Exemples

### Matériel et méthodes :

### Obtention des fucanes :

Les fucanes utilisés dans l'invention sont extraits de Phéophycées ou algues brunes (*Ascophyllum nodosum)* selon des procédés déjà décrits (selon Black et al, J. Sci. Food Agric., 3,122-129, 1952 ou selon Nishino et al. Carbohyd. Res., 186, 119-129, 1989). Les fucanes de bas poids moléculaire (FBPM) utilisés sont obtenus par des procédés déjà décrits : par hydrolyse ménagée suivie d'un fractionnement préparatif sur gel perméable ou d'exclusion stérique (brevet EP0403377) ou par dépolymérisation radicalaire (brevets EP 0 846 129 et EP1207891).

### Obtention des ostéoblastes :

Les cellules ostéoblastiques humaines proviennent de fragments osseux récupérés au bloc opératoire lors de réductions de fracture. Les prélèvements osseux sont rincés dans du PBS jusqu'à élimination des éléments figurés du ,sang. Des carottes osseuses sont découpées en cubes de 3 à 4 mm, puis déposées dans des boites de cultures. Une goutte de milieu de culture, (DMEM 40% de sérum de veau foetal (SVF), pénicilline (100 UI/ml), de la streptomycine (100 µg/ml), fungizone (2 µg/ml)) est déposé sur chaque explant pour permettre leur adhésion au plastique de la boite. La boite de culture est ensuite placée dans un incubateur (37°C, air (95%)/ CO₂ (5%)) pendant 1 à 2 heures. Les explants sont alors recouverts de milieu de culture DMEM comprenant 20% de SVF. Ce milieu est changé tous les 3 jours. Les cellules migrent hors de l'expiant pour coloniser la boite de culture dès le 6^{ème} jour de culture, la confluence est atteinte après 4 semaines de culture, les explants sont alors enlevés de.la boite de culture.

### Culture des ostéoblastes en deux dimensions

Les ostéoblastes confluents au 1^{er} passage de culture sont ensemencés à raison de 10000 cellules/ml dans des boites 24 puits puis incubés pendant 24 heures dans un milieu DMEM comprenant 10% de SVF additionné de pénicilline et de streptomycine. Après adhésion et étalement des cellules, les milieux de culture sont remplacés par des milieux DMEM comprenant 10% de SVF contenant ou non le fucane (10µg/ml). Ces milieux de culture sont ensuite changés tous les 3 jours. Au 21^{ème} jour de culture, afin de parfaire la différenciation ostéoblastique du β glycérophosphate (10 mM) et de l'acide ascorbique 2 phosphate (25 mM) sont ajoutés. Après 8, 15, 30, et 45 jours de culture, les surnageants de culture sont prélevés afin d'étudier la sécrétion des métalloprotéases matricielles. Les cellules sont trypsinisées pour les numérations cellulaires ou fixées à l'éthanol absolu (-20°C) ce qui permettra par la suite les études morphologiques et immunocytochimiques.

### Préparation du biomatériau pour la culture cellulaire

Le biomatériau est une trame osseuse spongieuse d'origine bovine obtenue par nettoyage des espaces inter-trabéculaire et par élimination des protéines non collagéniques. Le biomatériau est au préalable coupé en petits fragments (2mmx2mm),ces fragments sont laissés pendant 48 heures dans de l'éthanol absolu, puis incubés dans du DMEM pendant 24 heures à 37°C (air/5%CO₂) dans une étuve de culture. Les biomatériaux osseux sont réhydratés dans les milieux de culture en présence ou non de fucanes (50µg/ml).

### Culture des ostéoblastes dans le biomatériau osseux.

Les ostéoblastes humains normaux sont ensemencés dans des boites de culture 24 puits (20000 cellules/puits) contenant les biomatériaux osseux imprégnés ou non de fucanes. Ces greffons sont maintenus dans un milieu de culture dit minéralisant composé de DMEM, de sérum de veau foetal (10%), d'acide ascorbique (50 µg/ml), d'insuline (5µg/ml) de transferrine (5µg/ml). Durant toute la durée de l'expérimentation, ce milieu de culture est renouvelé tous les 3 jours. Les greffons ainsi obtenus sont retirés des boites de culture après 10 ou 30 jours de culture, fixés et préparés pour les études histologiques et en microscopie électronique à balayage.

### Coloration des cellules au GIEMSA

Cette coloration permet la visualisation du noyau et du cytoplasme. Après fixation, les cellules sont recouvertes pendant 2 minutes de colorant GIEMSA (MERCK) préalablement filtré. L'excès de colorant est éliminé par des rinçages successifs à l'eau distillée.

### Réaction de Von Kossa

Cette réaction permet de visualiser les dépôts minéraux dans les cultures. Ces dépôts apparaissant comme des zones noires. Les cultures bidimensionnelles après fixation sont rincées à l'eau distillée puis incubées pendant 30 min dans une solution de nitrate d'argent (5%). Après rinçage à l'eau distillée le matériel est exposé à la lumière solaire.

### Visualisation de l'activité Phosphatase alcaline in situ

Les cellules sont rincées au PBS puis incubées dans une solution tampon (Tris-HCL (0,05M) pH = 9,5, MgSO₄ (0,1%), Naphtyl phosphate (0,1%), Fast red (0,1%)) pendant 1 heure à température ambiante. Le surnageant est éliminé et les puits sont rincés à l'eau distillée 2 à 3 fois. Les zones à activité phosphatase alcaline positive apparaissent brunes.

### Immuno-détection du collagène de type I

Les cellules ou les coupes de biomatériau osseux sont rincées au PBS puis incubées dans une solution de CH₃OH (80%)/H₂O₂ (20%) pendant 10 minutes afin d'inactiver les peroxydases endogènes. Les puits ou les coupes sont rincées au PBS, puis les sites antigéniques non spécifiques sont bloqués (lait écrémé 0,1%, 10 min). Après rinçage le matériel est incubé avec un IgG de souris anti-collagène humain (Sigma, 1/40^{ème}) pendant 1 heure. Après rinçage le matériel subit une nouvelle incubation avec un anticorps de chèvre marqué à la peroxydase et dirigé contre les IgG de souris (1/60^{ème}) (calbiochem). Après rinçage, l'activité péroxydase des zones comportant du collagène de type I est révélée après réaction avec la 3-3'-diaminobenzidine tétrachlorhydrate (Sigma) (15 min à l'abri de la lumière).

### Microscopie électronique à balayage

Les biomatériaux osseux sont aux temps 10 et 30 jours fixés au paraformaldéhyde 4%. Après rinçage au PBS et une post fixation au tetroxyde d'osmium à 2% pendant 45 minutes, les biomatériaux sont rincés dans trois bains successifs de cacodylate de sodium, puis déshydratés par des solution d'éthanol croissantes jusqu'à l'éthanol absolu. La substitution de l'alcool par du CO₂ liquide est effectuée dans un appareil à point critique. Une métallisation à l'or de la surface de l'échantillon est effectuée par pulvérisation cathodique sous vide afin d'obtenir une couche de surface conductrice nécessaire à une observation optimale de l'échantillon en microscopie électronique à balayage.

### Résultats :

### Cultures bidimensionnelles

Les cellules issues d'explants osseux sont considérées comme des cellules pré-ostéoblastiques qui, en culture vont acquérir leur phénotype purement ostéoblastique. Cette différenciation terminale se fait après plusieurs phases, correspondant à une phase proliférative, une phase de synthèse matricielle et enfin une phase maturation et de minéralisation. La fin de la phase proliférative se caractérise après confluence par l'apparition de nodules composés d'ostéoblastes matures dans une matrice extracellulaire tridimensionnelle. La synthèse de collagène de type I est maximale au moment de la formation de ces nodules puis diminue rapidement. La phase de maturation est caractérisée par une augmentation de l'expression de la phosphatase alcaline (PAL) qui atteint son apogée au début de la phase de minéralisation. Puis, avec la différenciation terminale des cellules ostéoblastiques, l'expression de la PAL diminue.

### Prolifération des ostéoblastes en culture bidimensionnelle

L'addition de fucanes dans le milieu de culture stimule fortement la prolifération des cellules ostéoblastiques (+45% à 30 jours, +60% à 45 jours cf. Fig. 1)).

### Phosphatase alcaline

La cinétique d'expression de la phosphatase alcaline (PAL) par les ostéoblastes en culture permet d'apprécier l'avancement de ces cellules dans leurs voies de différenciation. L'apparition de la phosphatase alcaline dans les cultures marque le début de la différenciation ostéoblastique alors que les ostéoblastes matures n'expriment plus cette enzyme.
L'expression de la PAL dans les cultures témoins est observée dès 15 jours de culture (Fig. 2a) et est maximale à 45 jours (Fig. 2d), cette expression est beaucoup plus précoce dans les cultures incubées en présence de fucanes (10µg/ml) (cf. Fig. 3a, 3b, 3c). En effet en présence de fucanes, l'expression de la PAL est observée dès 8 jours de culture (Fig. 3a) atteint son maximum après 15 jours (Fig. 3b), pour avoir nettement diminuée dans les culture observées au 30^{ème} jour (Fig. 3c). L'observation de l'expression de la PAL n'est plus possible après 45 jours de culture en présence du fucanes, des dépôts minéraux couvrant la quasi totalité de la boite de culture.

### Réaction Von Kossa et immuno-détection du collagène de type I

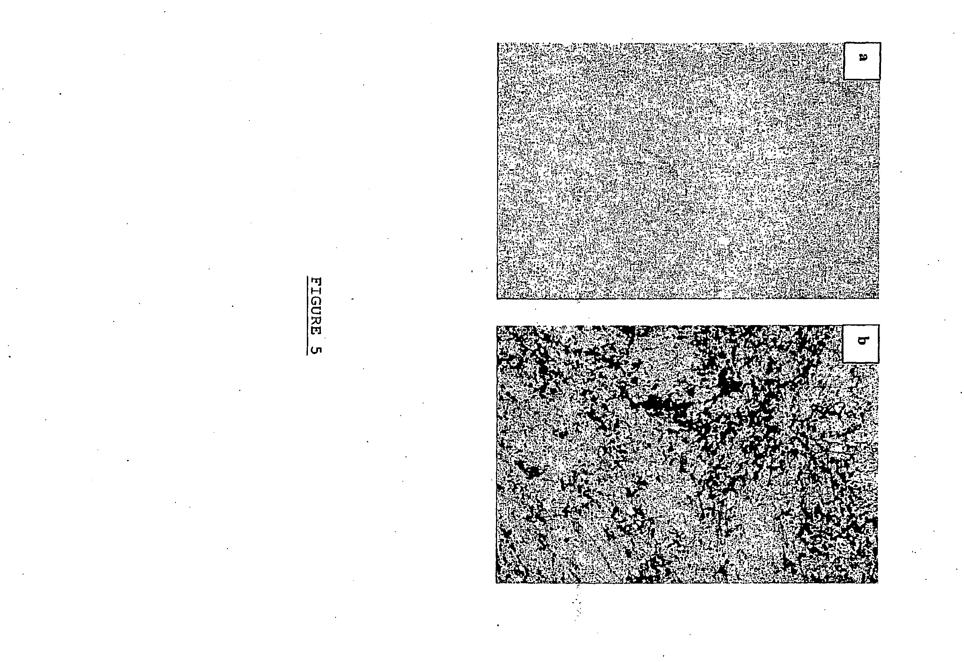

La réaction de Von Kossa permet d'apprécier l'état de minéralisation de la matrice extracellulaire sécrétée par les ostéoblastes en culture (Fig. 4). Quelques nodules de minéralisation sont détectés dans les cultures témoins à 45 jours (Fig. 4c), alors que ces mêmes nodules sont observés dès 30 jours dans les cultures en présence du fucanes (Fig. 4b). A 45 jours de culture, la matrice extracellulaire exprimée par les ostéoblastes incubés avec le polysaccharide est quasiment entièrement minéralisée (Fig. 4d).
Après 45 jours, la présence de collagène de type I est détectée au sein de la matrice extracellulaire des cultures témoins ou incubées par le fucane (Fig. 5). Le dépôt de ce collagène fibrillaire dans les cultures témoins forme un réseau peu ou pas minéralisé, étroitement associé avec les prolongements cellulaires (Fig. 5a). La détection du collagène I dans les cultures traitées par les fucanes montre une trame collagénique associée à un dépôt dense correspondant au dépôt minéral observé après réaction de Von Kossa (Fig. 5b).

### Expression de la gélatinase A (MMP-2) et de la collagénase 3 (MMP-13)

Les ostéoblastes en culture expriment la MMP-2 et la MMP-13, la MMP-2 est surtout exprimée en début de culture tandis que la MMP-13 est exprimée par les ostéoblastes en fin de différentiation. L'addition de fucanes à la culture semble diminuer l'expression de la gélatinase A par les ostéoblastes, tandis que l'expression de MMP-13 induite est le reflet de l'accélération de la différenciation des cellules dans la lignée ostéoblastique.

### Conclusion :

### L'addition de fucanes au milieu de culture accélère la différentiation de lignées ostéoblastiques.

### Culture tridimensionnelle

### Microscopie électronique à balayage:

La microscopie électronique à balayage a permis d'observer, la structure macromoléculaire des biomatériaux ainsi que leur colonisation par les ostéoblastes.

### Biomatériaux acellulaires

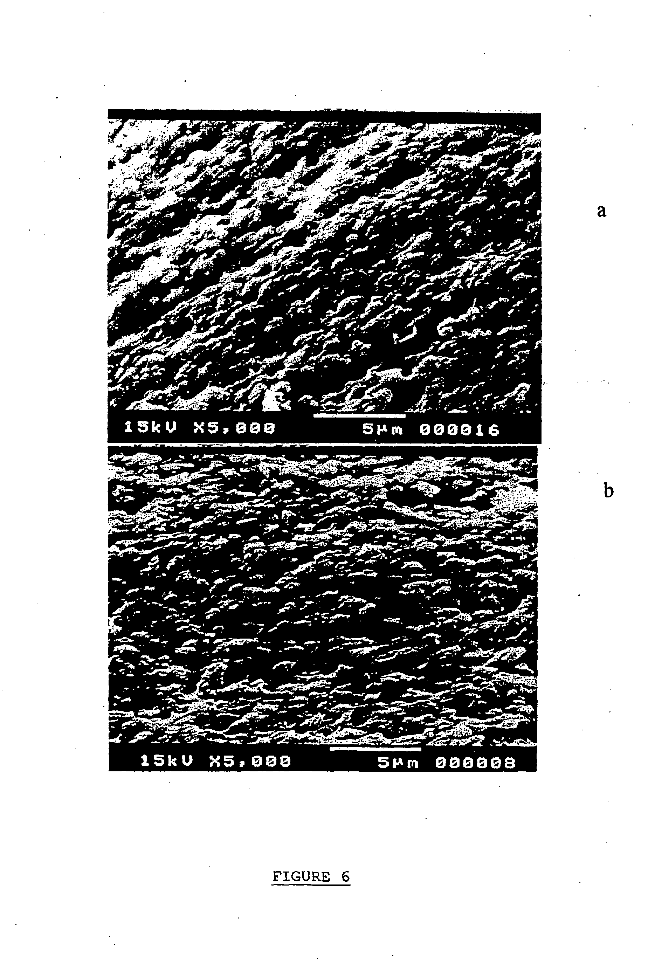

L'observation des biomatériaux non ensemencés par des ostéoblastes montre que le prétraitement par les fucanes ne modifie pas l'ultrastructure du biomatériau osseux (Fig. 6a, 6b).

### Biomatériaux ensemencés par des ostéoblastes

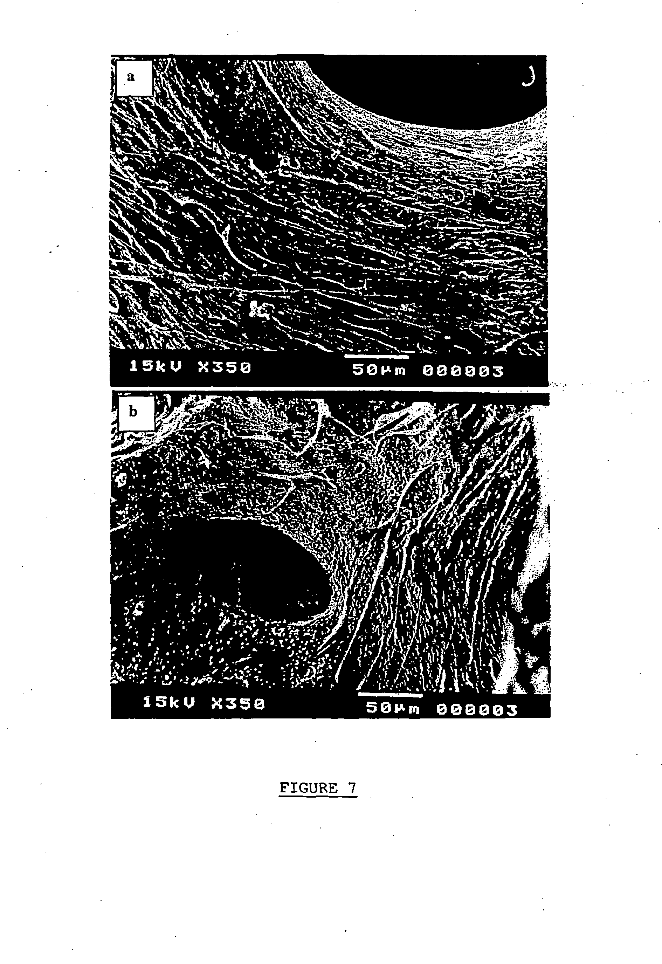

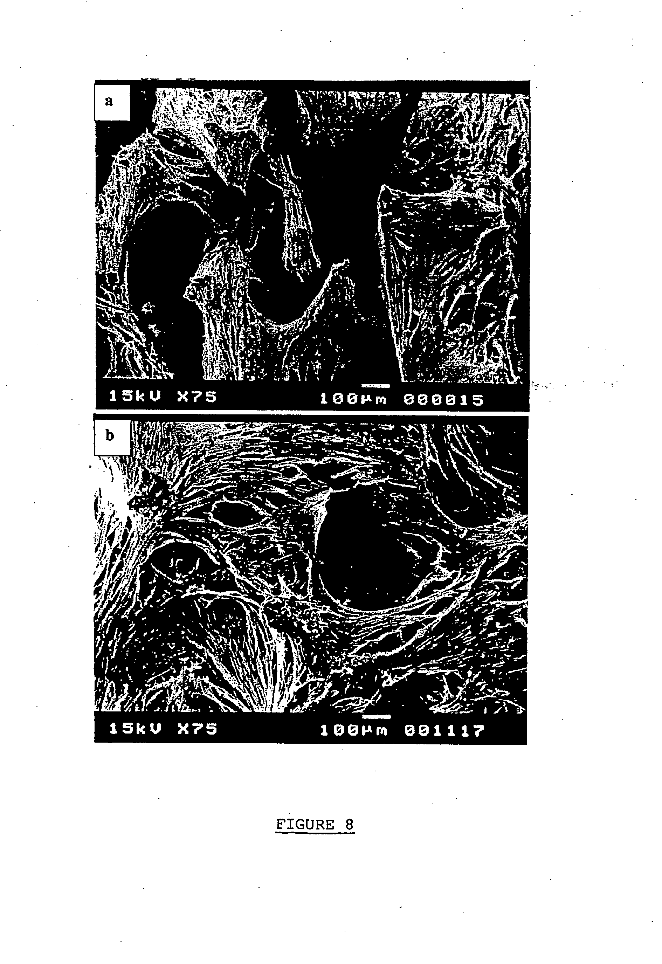

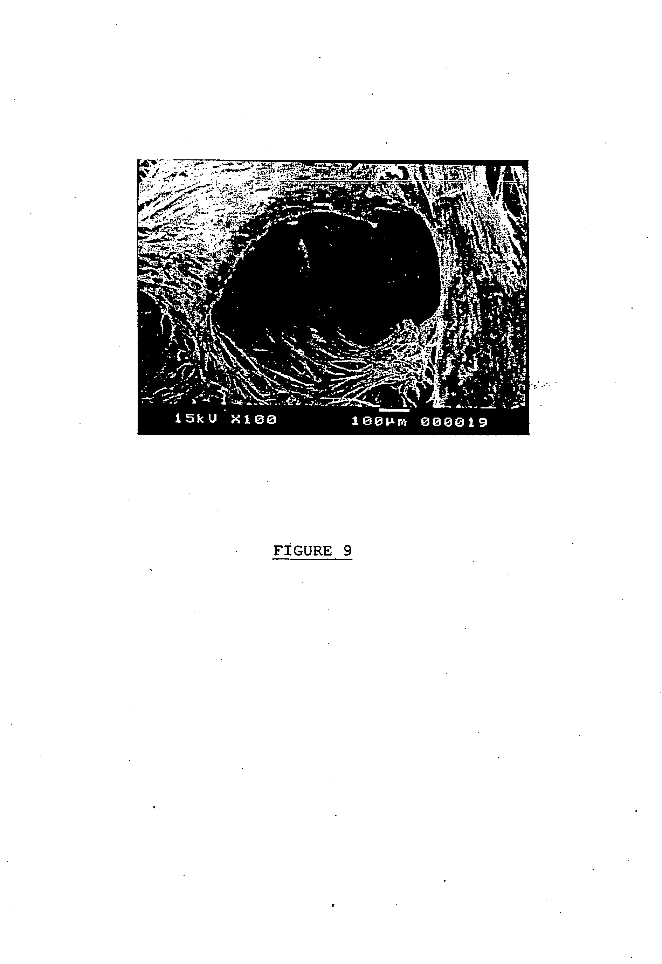

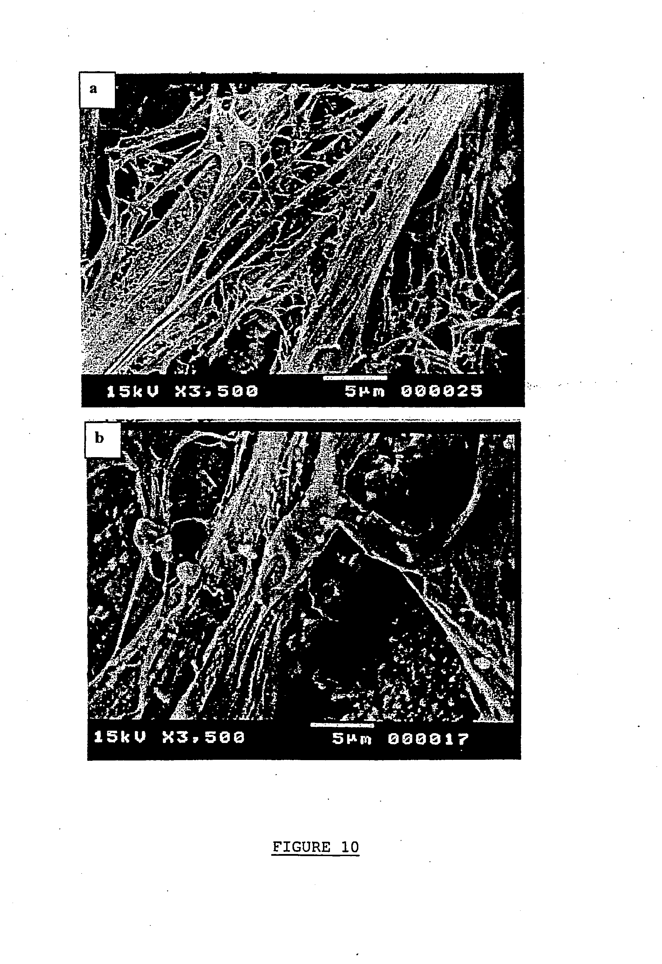

A 10 jours de culture les cellules sont adhérentes à la surface des biomatériaux imprégnés ou non par les fucanes. Ces cellules possèdent un corps cellulaire aplati, qui adhère à la trame collagénique, d'où partent des pseudopodes fins et allongés permettant de parfaire l'adhésion à la matrice extracellulaire (Fig. 7).
Après 30 jours de culture la cellularité des biomatériaux témoins et comprenant les fucanes est largement supérieure à celle observée après 10 jours, ce qui laisse supposer une prolifération des ostéoblastes au sein même de la matrice collagénique (Fig. 8). De plus à 30 jours de culture la densité cellulaire des biomatériaux comprenant les fucanes (Fig. 8b) est beaucoup plus importante que celle observée pour les biomatériaux non traités (Fig. 8a). En effet, sans fucane, les ostéoblastes (Fig. 8a), n'occupent pas tout le volume du biomatériau, les pores du biomatériau restent visibles. Après imprégnation par les fucanes, les biomatériaux présentent une forte densité cellulaire et les pores sont en grande partie obturés par les cellules (Fig. 8b). Les ostéoblastes colonisent en profondeur ces pores et forment des ponts de part et d'autre de leurs berges (Fig. 9).
Au contact des cellules, la présence de matériel filamentaire extracellulaire est observée. Cela correspond à la sécrétion par les ostéoblastes d'une matrice extracellulaire fibrillaire. D'autre part, on observe la présence d'un matériel globulaire extracellulaire en contact avec les ostéoblastes et la matrice extracellulaire filamentaire (Fig. 10b). Cela correspond à de la matrice calcifiée

### Conclusions

**L'imprégnation du biomatériau osseux par les fucanes ne modifie pas l'adhésion cellulaire.**
**L'imprégnation du biomatériau osseux par les fucanes stimule la prolifération des ostéoblastes.**
**L'imprégnation du biomatériau osseux par les fucanes accélère la minéralisation de la matrice extracellulaire sécrétée par les ostéoblastes.**

## Revendications

1. Substitut osseux présentant une activité sur la régénération osseuse, comprenant des fucanes présentant une masse molaire moyenne en poids comprise entre 5 000 et 100 000 g/mol, en particulier comprise entre 10 000 et 40 000 g/mol.

2. Substitut osseux selon la revendication **1**, **caractérisé en ce que** les fucanes sont obtenus à partir d'algue brune.

3. Substitut osseux selon l'une quelconques des revendications **1** à **2**, comprenant un biomatériau.

4. Substitut osseux selon l'une quelconques des revendications **1** à **3**, **caractérisé en ce que** le biomatériau comprend un ou plusieurs matériau(x) choisi(s) dans le groupe constitué par le titane, le collagène, l'os déprotéinisé et/ou déminéralisé, le corail, la céramique en phosphate de calcium, l'hydroxyapatite, le phosphate tricalcique beta et les verres bioactifs.

5. Substitut osseux selon l'une quelconque des revendications **1** à **4**, caractérisé en qu'il comprend en outre un ou plusieurs facteurs de croissance choisis dans le groupe constitué par Fibroblast Growth Factors (FGFs), Transforming Growth Factors (TGFs), Insulin Growth Factors I (IGFs), Platelet Derived Growth Factors (PDGFs), Bone Morphogenetic Proteins (BMPs) et Vascular Endothelial Growth Factors (VEGFs).

6. Substitut osseux selon l'une quelconque des revendications **1** à **5**, caractérisé en qu'il comprend en outre une ou plusieurs cytokines choisie(s) dans le groupe constitué par l'interleukine 1, l'interleukine 4, l'interleukine 6, le Tumor Necrosis Factor alpha, le Granulocyte-Macrophage Colony-Stimulating Factor et le Macrophage Colony-Stimulating Factor.

7. Substitut osseux selon l'une quelconque des revendications **1** à **6**, **caractérisé en ce que** la surface du dit biomatériau présente un revêtement comprenant lesdits fucans.

8. Substitut osseux selon l'une quelconque des revendications **1** à **7**, **caractérisé en ce que** le dit biomatériau est imprégné avec lesdits fucanes.

9. Substitut osseux selon l'une quelconque des revendications **1** à **8**, caractérisé en qu'il comprend en outre des cellules ostéocompétentes issues de la moelle osseuse, de l'os ou du périoste.

10. Procédé de préparation de substitut osseux selon la revendication **7**, **caractérisé en ce que** la surface dudit biomatériau est recouverte par un revêtement comprenant lesdits fucanes.

11. Procédé de préparation de substitut osseux selon la revendication **8**, **caractérisé en ce que** le dit biomatériau est imprégné avec lesdits fucanes.

12. Procédé de préparation de substitut osseux selon la revendication **9**, **caractérisé en ce qu'**on procède à la colonisation d'un substitut osseux selon l'une quelconque des revendications **1** à **8**, par des cellules ostéocompétentes issues de la moelle osseuse, de l'os ou du périoste.

13. Procédé selon la revendication **12**, **caractérisé en ce que** les cellules ostéocompétentes issues de la moelle osseuse, de l'os ou du périoste sont cultivées dans un milieu de culture comprenant des fucanes présentant une masse molaire moyenne en poids comprise entre 5 000 et 100 000 g/mol, en particulier comprise entre 10 000 et 40 000 g/mol.

14. Milieu de culture pour un type de cellule choisi dans le groupe constitué de cellules ostéocompétentes issues de la moelle osseuse, de l'os ou du périoste, comprenant des fucanes présentant une masse molaire moyenne en poids comprise entre 5 000 et 100 000 g/mol, en particulier comprise entre 10 000 et 40 000 g/mol.

15. Milieu de culture selon la revendication **14**, **caractérisé en ce qu'**il comprend en outre un ou plusieurs facteurs de croissance choisis dans le groupe constitué par Fibroblast Growth Factors (FGFs), Transforming Growth Factors (TGFs), Insulin Growth Factors I (IGFs), Platelet Derived Growth Factors (PDGFs), Bone Morphogenetic Proteins (BMPs) et Vascular Endothelial Growth Factors (VEGFs).

16. Procédé de culture de cellules choisies dans le groupe des cellules ostéocompétentes issues de la moelle osseuse, de l'os ou du périoste, **caractérisé en ce que** lesdites cellules sont cultivées dans le milieu de culture selon la revendication **15** ou **16**.

17. Procédé de culture de cellules choisies dans le groupe des cellules ostéocompétentes issues de la moelle osseuse, de l'os ou du périoste, **caractérisé en ce que** lesdites cellules sont cultivées sur le substitut osseux selon l'une quelconque des revendications **1** à **9**.

18. Dispositif médical présentant une activité sur la régénération osseuse, comprenant des fucanes présentant une masse molaire moyenne en poids comprise entre 5 000 et 100 000 g/mol, en particulier comprise entre 10 000 et 40 000 g/mol.

19. Dispositif médical selon la revendication **18**, comprenant un ou plusieurs facteurs de croissance choisi(s) dans le groupe constitué par Fibroblast Growth Factors (FGFs), Transforming Growth Factors (TGFs), Insulin Growth Factor I (IGFs), Platelet Derived Growth Factors (PDGFs), Bone Morphogenetic Proteins (BMPs) et Vascular Endothelial Growth Factors (VEGFs).

20. Dispositif médical selon la revendication **18** ou la revendication **19**, comprenant un substitut osseux tel que défini dans l'une des revendications **1** à **9**.

## Patentansprüche

1. Knochenersatz mit Auswirkungen auf die Knochenregeneration, umfassend Fukane mit einem mittleren Molekulargewicht zwischen 5 000 und 100 000 g/mol, insbesondere zwischen 10 000 und 40 000 g/mol.

2. Knochenersatz nach Anspruch **1**, **dadurch gekennzeichnet, dass** die Fukane aus Braunalgen gewonnen sind.

3. Knochenersatz nach einem der Ansprüche **1** bis **2**, welcher ein Biomaterial umfasst.

4. Knochenersatz nach einem der Ansprüche **1** bis **3**, **dadurch gekennzeichnet, dass** das Biomaterial eines oder mehrere Materialien umfasst, die gewählt sind aus der Gruppe, umfassend Titan, Kollagen, entproteinisierter und/oder entmineralisierter Knochen, Koralle, Keramik aus Kalziumphosphat, Hydroxyapatit, beta-Trikalziumphosphat und bioaktives Glas.

5. Knochenersatz nach einem der Ansprüche **1** bis **4**, **dadurch gekennzeichnet, dass** dieser unter anderem einen oder mehrere Wachstumsfaktoren umfasst, die gewählt sind aus der Gruppe, bestehend aus Fibroblast Growth Factors (FGFs), Transforming Growth Factors (TGFs), Insulin Growth Factors I (IGFs), Platelet Derived Growth Factors (PDGFs), Bone Morphogenetic Proteins (BMPs) und Vascular Endothelial Growth Factors (VEGFs).

6. Knochenersatz nach einem der Ansprüche **1** bis **5**, **dadurch gekennzeichnet, dass** er unter anderem ein oder mehrere Zytokine umfasst, gewählt aus der Gruppe, umfassend Interleukin 1, Interleukin 4, Interleukin 6, Tumor Necrosis Factor alpha, Granulocyte-Macrophage Colony-Stimulating Factor und Macrophage Colony-Stimulating Factor.

7. Knochenersatz nach einem der Ansprüche **1** bis **6**, **dadurch gekennzeichnet, dass** die Oberfläche des Biomaterials eine Beschichtung aufweist, die die Fukane umfasst.

8. Knochenersatz nach einem der Ansprüche **1** bis **7**, **dadurch gekennzeichnet, dass** das Biomaterial mit den Fukanen durchtränkt ist.

9. Knochenersatz nach einem der Ansprüche **1** bis **8**, **dadurch gekennzeichnet, dass** dieser ferner osteokompetente Zellen umfasst, die aus dem Knochenmark, dem Knochen oder dem Periost stammen.

10. Herstellungsverfahren für Knochenersatz nach Anspruch **7**, **dadurch gekennzeichnet, dass** die Oberfläche des Biomaterials von einer Beschichtung bedeckt ist, die die Fukane umfasst.

11. Herstellungsverfahren für Knochenersatz nach Anspruch **8**, **dadurch gekennzeichnet, dass** das Biomaterial mit den Fukanen durchtränkt wird.

12. Herstellungsverfahren für Knochenersatz nach Anspruch **9**, **dadurch gekennzeichnet, dass** eine Kolonisierung des Knochenersatzes nach einem der Ansprüche **1** bis **8** durch osteokompetente Zellen vorgenommen wird, die aus dem Knochenmark, dem Knochen oder dem Periost stammen.

13. Verfahren nach Anspruch **12**, **dadurch gekennzeichnet, dass** die osteokompetenten Zellen, die aus dem Knochenmark, dem Knochen oder dem Periost stammen, in einem Kulturmedium kultiviert werden, das Fukane mit einem mittleren Molekulargewicht zwischen 5 000 und 100 000 g/mol, insbesondere zwischen 10 000 und 40 000 g/mol, umfasst.

14. Kulturmedium für einen Zellentyp, der gewählt ist aus der Gruppe, bestehend aus osteokompetenten Zellen aus dem Knochenmark, dem Knochen oder dem Periost, umfassend Fukane mit einem mittleren Molekulargewicht zwischen 5 000 und 100 000 g/mol, insbesondere zwischen 10 000 und 40 000 g/mol.

15. Kulturmedium nach Anspruch **14**, **dadurch gekennzeichnet, dass** dieses ferner einen oder mehrere Wachstumsfaktoren umfasst, die aus der Gruppe, bestehend aus Fibroblast Growth Factors (FGFs), Transforming Growth Factors (TGFs), Insulin Growth Factors I (IGFs), Platelet Derived Growth Factors (PDGFs), Bone Morphogenetic Proteins (BMPs) und Vascular Endothelial Growth Factors (VEGFs), gewählt sind.

16. Verfahren zur Kultivierung von Zellen, die aus der Gruppe von osteokompetenten Zellen aus dem Knochenmark, dem Knochen oder dem Periost gewählt werden, **dadurch gekennzeichnet, dass** die Zellen in dem Kulturmedium nach Anspruch **15** oder **16** kultiviert werden.

17. Verfahren zur Kultivierung von Zellen, die aus der Gruppe von osteokompetenten Zellen aus dem Knochenmark, dem Knochen oder dem Periost gewählt werden, **dadurch gekennzeichnet, dass** die Zellen auf dem Knochenersatz nach einem der Ansprüche **1** bis **9** kultiviert werden.

18. Medizinische Vorrichtung, die eine Auswirkung auf die Knochenregeneration aufweist, umfassend Fukane mit einem mittleren Molekulargewicht zwischen 5 000 und 100 000 g/mol, insbesondere zwischen 10 000 und 40 000 g/mol.

19. Medizinische Vorrichtung nach Anspruch **18**, welche einen oder mehrere Wachstumsfaktoren umfasst, die aus der Gruppe, bestehend aus Fibroblast Growth Factors (FGFs), Transforming Growth Factors (TGFs), Insulin Growth Factors I (IGFs), Platelet Derived Growth Factors (PDGFs), Bone Morphogenetic Proteins (BMPs) und Vascular Endothelial Growth Factors (VEGFs), gewählt sind.

20. Medizinische Vorrichtung nach Anspruch **18** oder Anspruch **19**, welche einen Knochenersatz gemäß der Definition in einem der Ansprüche 1 bis **9** umfasst.

## Claims

1. Bone substitute having an activity on bone regeneration, comprising fucans with a weight-average molar mass of between 5000 and 100 000 g/mol, in particular between 10 000 and 40 000 g/mol.

2. Bone substitute according to Claim **1**, **characterized in that** the fucans are obtained from a brown alga.

3. Bone substitute according to either one of Claims **1** and **2**, comprising a biomaterial.

4. Bone substitute according to any one of Claims **1** to **3**, **characterized in that** the biomaterial comprises one or more material(s) chosen from the group consisting of titanium, collagen, deproteinated and/or demineralized bone, coral, calcium phosphate ceramic, hydroxyapatite, beta-tricalcium phosphate and bioactive glasses.

5. Bone substitute according to any one of Claims **1** to **4**, **characterized in that** it further comprises one or more growth factors chosen from the group consisting of fibroblast growth factors (FGFs), transforming growth factors (TGFs), insulin growth factors I (IGFs), platelet derived growth factors (PDGFs), bone morphogenetic proteins (BMPs) and vascular endothelial growth factors (VEGFs).

6. Bone substitute according to any one of Claims **1** to **5**, **characterized in that** it further comprises one or more cytokines chosen from the group consisting of interleukin 1, interleukin 4, interleukin 6, tumor necrosis factor-alpha, granulocyte-macrophage colony-stimulating factor and macrophage colony-stimulating factor.

7. Bone substitute according to any one of Claims **1** to **6**, **characterized in that** the surface of said biomaterial has a coating comprising said fucans.

8. Bone substitute according to any one of Claims **1** to **7**, **characterized in that** said biomaterial is impregnated with said fucans.

9. Bone substitute according to any one of Claims **1** to **8**, **characterized in that** it further comprises osteocompetent cells derived from bone marrow, from bone or from the periosteum.

10. Method for preparing bone substitute according to Claim **7**, **characterized in that** the surface of said biomaterial is covered with a coating comprising said fucans.

11. Method for preparing bone substitute according to Claim **8**, **characterized in that** said biomaterial is impregnated with said fucans.

12. Method for preparing bone substitute according to Claim **9**, **characterized in that** the colonization of a bone substitute according to any one of Claims **1** to **8**, with osteocompetent cells derived from bone marrow, from bone or from the periosteum, is performed.

13. Method according to Claim **12**, **characterized in that** the osteocompetent cells derived from bone marrow, from bone or from the periosteum are cultured in a culture medium comprising fucans with a weight-average molar mass of between 5000 and 100 000 g/mol, in particular between 10 000 and 40 000 g/mol.

14. Culture medium for a cell type chosen from the group consisting of osteocompetent cells derived from bone marrow, from bone or from the periosteum, comprising fucans with a weight-average molar mass of between 5000 and 100 000 g/mol, in particular between 10 000 and 40 000 g/mol.

15. Culture medium according to Claim **14**, **characterized in that** it further comprises one or more growth factors chosen from the group consisting of fibroblast growth factors (FGFs), transforming growth factors (TGFs), insulin growth factors I (IGFs), platelet derived growth factors (PDGFs), bone morphogenetic proteins (BMPs) and vascular endothelial growth factors (VEGFs).

16. Method for culturing cells chosen from the group of osteocompetent cells derived from bone marrow, from bone or from the periosteum, **characterized in that** said cells are cultured in the culture medium according to Claim **15** or **16.**

17. Method for culturing cells chosen from the group of osteocompetent cells derived from bone marrow, from bone or from the periosteum, **characterized in that** said cells are cultured on the bone substitute according to any one of Claims **1** to **9**.

18. Medical device having an activity on bone regeneration, comprising fucans with a weight-average molar mass of between 5000 and 100 000 g/mol, in particular between 10 000 and 40 000 g/mol.

19. Medical device according to Claim **18**, comprising one or more growth factors chosen from the group consisting of fibroblast growth factors (FGFs), transforming growth factors (TGFs), insulin growth factors I (IGFs), platelet derived growth factors (PDGFs), bone morphogenetic proteins (BMPs) and vascular endothelial growth factors (VEGFs).

20. Medical device according to Claim **18** or Claim **19**, comprising a bone substitute as defined in one of Claims **1** to **9**.
